# EUROPEAN PATENT SPECIFICATION

(11) **EP 2 212 700 B1**
(45) Date of publication and mention of the grant of the patent: **24.08.2011**
(21) Application number: 08851789.1
(22) Date of filing: 18.11.2008
(51) Int. Cl.: G01N 33/574

(54) **METHOD OF ASSESSING COLORECTAL CANCER FROM A STOOL SAMPLE BY USE OF THE MARKER COMBINATION CALPROTECTIN AND HEMOGLOBIN/HAPTOGLOBIN COMPLEX**
VERFAHREN ZUR BEURTEILUNG VON KOLOREKTALEM KREBS ANHAND EINER STUHLPROBE DURCH VERWENDUNG EINER MARKER-KOMBINATION AUS CALPROTECTIN UND EINEM HÄMOGLOBIN/HAPTOGLOBIN-KOMPLEX
PROCÉDÉ DE DÉTECTION DU CANCER COLORECTAL À PARTIR D'UN ÉCHANTILLON DE SELLES AU MOYEN D'UNE COMBINAISON DE MARQUEURS ASSOCIANT LA CALPROTECTINE ET LE COMPLEXE HÉMOGLOBINE/HAPTOGLOBINE

(30) Priority: 20.11.2007 EP 07022448
(43) Date of publication of application: 04.08.2010
(73) Proprietor: Roche Diagnostics GmbH, 68305 Mannheim (DE); F.Hoffmann-La Roche AG, 4070 Basel (CH)
(72) Inventor: KARL, Johann, 82380 Peissenberg (DE); GARCZAREK, Ursula, 44309 Dortmund (DE); WILD, Norbert, 82538 Geretsried (DE)
(86) International application number: PCT/EP2008/009733
(87) International publication number: WO 2009/065551

(56) References cited:
- WO-A-98/20355
- WO-A-2007/134779
- JP-A- 2004 251 851
- HOFF G ET AL: "Testing for faecal calprotectin (PhiCal) in the Norwegian Colorectal Cancer Prevention trial on flexible sigmoidoscopy screening: comparison with an immunochemical test for occult blood (FlexSure OBT)" September 2004 (2004-09), GUT, VOL. 53, NR. 9, PAGE(S) 1329-1333 , XP000910339 ISSN: 0017-5749 the whole document
- ROSETH A G ET AL: "ASSESSMENT OF THE NEUTROPHIL DOMINATING PROTEIN CALPROTECTIN IN FECES A METHODOLOGIC STUDY" 1992, SCANDINAVIAN JOURNAL OF GASTROENTEROLOGY, VOL. 27, NR. 9, PAGE(S) 793-798 , XP009110340 ISSN: 0036-5521 the whole document
- LIMBURG PAUL J ET AL: "Prospective evaluation of fecal calprotectin as a screening biomarker for colorectal neoplasia." AMERICAN JOURNAL OF GASTROENTEROLOGY, vol. 98, no. 10, October 2003 (2003-10), pages 2299-2305, XP002509148 ISSN: 0002-9270
- GILBERT J A ET AL: "FECAL MARKER VARIABILITY IN COLORECTAL CANCER: CALPROTECTIN VERSUS HEMOGLOBIN" 1 January 1996 (1996-01-01), SCANDINAVIAN JOURNAL OF GASTROENTEROLOGY, XX, XX, PAGE(S) 1001 - 1005 , XP002053475 ISSN: 0036-5521 the whole document
- SIEG A ET AL: "DETECTION OF COLORECTAL NEOPLASMS BY THE HIGHLY SENSITIVE HEMOGLOBIN-HAPTOGLOBIN COMPLEX IN FECES" 1 December 1999 (1999-12-01), INTERNATIONAL JOURNAL OF COLORECTAL DISEASE, SPRINGER VERLAG, BERLIN, DE, PAGE(S) 267 - 271 , XP001182520 ISSN: 0179-1958 the whole document
- KARL JOHANN ET AL: "Improved diagnosis of colorectal cancer using a combination of fecal occult blood and novel fecal protein markers." CLINICAL GASTROENTEROLOGY AND HEPATOLOGY : THE OFFICIAL CLINICAL PRACTICE JOURNAL OF THE AMERICAN GASTROENTEROLOGICAL ASSOCIATION OCT 2008, vol. 6, no. 10, October 2008 (2008-10), pages 1122-1128, XP009110343 ISSN: 1542-7714

## Description

The present invention relates to a method aiding in the assessment of colorectal cancer. The method especially is used in assessing the absence or presence of colorectal cancer in vitro. The method is for example practiced by analyzing biochemical markers, comprising measuring in a stool sample the concentration of the hemoglobin/haptoglobin complex and calprotectin and correlating the concentrations determined to the absence or presence of colorectal cancer. To further improve the assessment of colorectal cancer based on a method of this invention the level of one or more additional marker may be determined together with the hemoglobin/haptoglobin complex and calprotectin in a stool sample and be correlated to the absence or presence of colorectal cancer. The invention also relates to the use of a marker panel comprising the hemoglobin/haptoglobin complex and calprotectin in the early diagnosis of colorectal cancer and it teaches a kit for performing the method of the invention.

### Background of the Invention

Cancer remains a major public health challenge despite progress in detection and therapy. Amongst the various types of cancer, colorectal cancer (= CRC) is one of the most frequent cancers in the Western world.

The staging of cancer is the classification of the disease in terms of extent, progression, and severity. It groups cancer patients so that generalizations can be made about prognosis and the choice of therapy.

Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds), TNM Classification of Malignant Tumours, fifth edition, 1997.

What is especially important is that early diagnosis of CRC translates to a much better prognosis. Malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

A method of assessing the presence or absence of CRC is looked for that is especially appropriate for the sensitive detection of CRC at a pre-malignant state (adenoma) or at a tumor stage where no metastases at all (neither proximal nor distal), i.e. in UICC classes I, II, or III, are present.

The earlier cancer can be detected/diagnosed; the better is the overall survival rate. This is especially true for CRC. The prognosis in advanced stages of tumor is poor. More than one third of the patients will die from progressive disease within five years after diagnosis, corresponding to a survival rate of about 40% for five years. Current treatment is only curing a fraction of the patients and clearly has the best effect on those patients diagnosed in an early stage of disease.

With regard to CRC as a public health problem, it is essential that more effective screening and preventative measures for colorectal cancer be developed.

The earliest detection procedures available at present for colorectal cancer involve using tests for fecal blood or endoscopic procedures. However, significant tumor size must typically exist before fecal blood is detected. With regard to detection of CRC from a stool sample, the state of the art has been for quite a while the guaiac-based fecal occult blood test.

The guaiac test is currently most widely used as a screening assay for CRC from stool. The guaiac test, however, has both poor sensitivity as well as poor specificity. The sensitivity of the guaiac-based fecal occult blood tests is ~ 26%, which means 74% of patients with malignant lesions will remain undetected (Ahlquist, D.A., Gastroenterol. Clin. North Am. 26 (1997) 41-55).

The visualization of precancerous and cancerous lesions represents the best approach to early detection, but colonoscopy is invasive with significant costs, risks, and complications (Silvis, S.E., et al., JAMA 235 (1976) 928-930; Geenen, J.E., et al., Am. J. Dig. Dis. 20 (1975) 231-235; Anderson, W.F., et al., J. Natl. Cancer Institute 94 (2002) 1126-1133).

Stool or fecal samples are routinely tested for the presence of parasites, fat, occult blood, viruses, bacteria and other organisms and chemicals in the diagnosis for various diseases.

Stool collection is non-invasive and thus theoretically ideal for testing pediatric or geriatric patients, for testing away from a clinical site, for frequently repeated tests and for determining the presence of analytes which are likely to be found in the digestive tract.

The diagnostic method according to the present invention is based on a stool sample which is derived from an individual. The stool sample is extracted and the hemoglobin/haptoglobin complex and calprotectin, respectively, is specifically measured from this processed stool sample by use of a specific binding agent.

However, the application of immuno assay techniques to analysis of fecal samples has proven to be difficult for several reasons.

Analytes are not distributed throughout the stool specimen but tend to be more concentrated at the outer surface of stool specimen that previously has been in contact with intestinal or even cancerous cells. This is why EP 0 817 968 proposes the use of cross-sectional stool sample for further analysis. The focus of EP 0 817 968 lies in the diagnosis of DNA as comprised in a stool specimen.

Stool handling is disagreeable and biohazardous. Procedures for processing stool have proven to be awkward and frequently complex requiring several handling steps, e.g., filtration or centrifugation. Weighing, extracting, centrifuging, and storing samples are difficult except in a clinical laboratory equipped with suitable apparatuses and skilled technicians.

Analytes in stool samples are frequently unstable; this is believed to be especially true for polypeptides or proteins. Constituents of stool are known to interfere with solid-phase immuno assays. Immunoreactants immobilized on solid-phase may be desorbed by stool constituents. Non-specific reactions may occur.

To increase the commercial use of immuno assay techniques for measuring a proteinaceous analyte in a stool sample, a number of problems must be solved. E.g. analytes have to be solubilized as efficient as possible, the instability of the analyte in the stool has to be dealt with, the interference from stool constituents should be reduced as much as possible, the needs for extensive handling of the stool, equipment contamination, and instrumentation needs must be minimized. Simple preparation steps avoiding the use of expensive equipment and instruments are required to extend the use of immunoassay testing procedures, or at least the sampling procedure for such immunoassay to sites outside hospital and clinical laboratory environments. Examples of stool sample diluents which are of advantage in the detection of proteins like the hemoglobin/haptoglobin complex and calprotectin are given further below.

WO 02/18931 discloses a method for preparing stool specimens for diagnostic assays. An improved extraction procedure based on an extraction buffer that essentially comprises a buffer substance, a detergent, preferably a zwitterionic detergent, and a blocking agent is described.

The handling of a stool specimen is facilitated by use of recently developed sampling devices. Appropriate stool sampling devices are e.g. described in EP 1 366 715 and in EP 1 214 447.

Despite the fact that immunological assays for proteins comprised in a stool specimen have been described since the early 1990ies, such assays still are not broadly used in clinical routine. US 5,198,365, for example, describes that it is possible to detect the presence of blood in a stool sample via the specific immunological measurement of hemoglobin.

A further alternative method to the guaiac test for detection of CRC in stool has been published recently and consists in the detection of the colorectal cancer-specific antigen, "minichromosome maintenance protein 2" (MCM2) by immunohistochemistry in colonic cells shed into stool. Due to the small study size, conclusion on the diagnostic value for detection of colorectal cancer is preliminary. However, the test seems to have only limited sensitivity to detect right-sided colon cancer (Davies, R.J., et al., Lancet 359 (2002) 1917-1919).

Calprotectin has been described as an alternative biomarker for the detection of CRC from stool samples in US 5,455,160 and correspondingly in the scientific literature by Roseth, A.G., et al. (Scand. J. Gastroentero.l 27 (1992) 793-798; Scand. J. Gastroenterol. 28 (1993) 1073-1076). Although calprotectin is a marker of inflammatory diseases its potential as a marker for the detection of CRC from stool is documented by several publications (Johne, B., et al., Scand. J. Gastroenterol. 36 (2001) 291-296; Limburg, P.J., et al., Am. J. Gastroenterol. 98 (2003) 2299-2305; Hoff, G., et al., Gut 53 (2004) 1329-1333). While the sensitivity and specificity of calprotectin is lower compared to the immunological hemoglobin-haptoglobin complex, calprotectin appears to have some characteristics favorable for a diagnostic biomarker as compared to hemoglobin or hemoglobin-haptoglobin-complex. It is homogeneously distributed in feces, it is stable at room temperature making mail delivery of the sample to the laboratory feasible and it shows no interference with food components or pharmaceutical compounds (Ton, H., et al., Clin. Chim. Acta 292 (2000) 41-54). However, elevated concentrations of calprotectin, the heterodimer of S100A8 and S100A9, were also detected in stool samples from patients suffering from Crohn's disease or inflammatory bowel disease. These results are in agreement with the more general role of calprotectin in inflammation (Ryckman, C., et al., J. Immunol. 170 (2003) 3233-3242). Hence, the use of calprotectin in gastroenterology is not limited to the detection of CRC but extends to other diseases, especially inflammatory bowel disease as reviewed by Poullis, A., et al. (J. Gastroenterol. Hepatol. 18 (2003) 756-762). Calprotectin would thus appear not to be a specific marker for CRC.

The sensitivity and specificity of diagnostic alternatives to the guaiac test have been recently investigated by Sieg, A., et al., Int. J. Colorectal Dis. 14 (1999) 267-271. Especially the measurement of hemoglobin and of the hemoglobin-haptoglobin complex from stool specimen have been compared. It has been noted that the hemoglobin assay has an unsatisfactory sensitivity for the detection of a colorectal neoplasm. Whereas cancer in its progressed carcinoma stage is detected with a sensitivity of about 87% the earlier tumor stages are not detected with a sufficient sensitivity. The hemoglobin-haptoglobin complex assay was more sensitive in the detection of earlier stages of CRC. However, this more sensitive detection was accompanied by a poor specificity. A poor specificity in a screening assay unfortunately translates to a high number of unnecessary secondary investigations, like colonoscopy, an assay with a poor accuracy also does not meet the requirements of a generally accepted screening assay.

A need clearly exists to improve the early detection and assessment of colorectal cancer.

It was the task of the present invention to find out whether the assessment of CRC, e.g. by use of immunological methods for detection of analytes in a stool specimen can be improved.

It has been found and established that a method for assessing the absence or presence of colorectal cancer in vitro by biochemical markers, comprising measuring in a stool sample the concentration of at least the combination of the hemoglobin/haptoglobin complex and calprotectin can help to overcome at least some of the disadvantages mentioned above. These findings are surprising since both individual markers appear to suffer from problems in specificity and also, because the marker combination of hemoglobin, which usually is a more specific marker for CRC, and calprotectin showed less sensitivity both at 95% specificity as well as at 98% specificity as compared to marker combination now identified.

### Summary of the Invention

The present invention relates to a method for assessing the absence or presence of colorectal cancer in vitro by biochemical markers, comprising measuring in a stool sample the polypeptide concentrations of at least the hemoglobin/haptoglobin complex and of calprotectin, respectively, and correlating the concentrations determined for the hemoglobin/haptoglobin complex and calprotectin to the absence or presence of colorectal cancer.

Further the use of a marker panel comprising at least the markers calprotectin and the hemoglobin/haptoglobin complex in the diagnosis of colorectal cancer is disclosed.

Also disclosed is a kit for performing the method according to the present invention comprising the reagents required to specifically measure the hemoglobin/haptoglobin complex and calprotectin, respectively, and optionally auxiliary reagents for performing the respective measurement.

### Detailed Description of the Invention

In a first embodiment the present invention relates to a method for assessing the absence or presence of colorectal cancer in vitro by biochemical markers, comprising measuring in a stool sample the concentration of at least (a) the hemoglobin/haptoglobin complex and (b) calprotectin, and (c) correlating the concentrations determined in steps (a) and (b) to the absence or presence of colorectal cancer.

The term "assessing" is used to indicate that the method according to the present invention will not alone but together with other variables, e.g., the confirmation by colonoscopy aid the physician to establish his diagnosis of colorectal cancer (CRC) or to draw his therapy-related conclusions. In a preferred embodiment this assessment will relate to the presence or absence of CRC. As the skilled artisan will appreciate no single biochemical marker and no marker combination is diagnostic with 100% specificity and at the same time 100% sensitivity for a given disease, rather biochemical markers are used to assess with a certain likelihood or predictive value the presence or absence of a disease. Preferably the method according to the present invention aids in assessing the presence or absence of CRC.

As the skilled artisan will appreciate the step of correlating a marker level to the presence or absence of CRC can be performed and achieved in different ways. In general a reference population is selected and a normal range established. It is no more than routine experimentation, to establish the normal range for both the hemoglobin/haptoglobin complex as well as for calprotectin in stool samples by using an appropriate reference population. It is generally accepted that the normal range to a certain but limited extent depends on the reference population in which it is established. The ideal and preferred reference population is high in number, e.g., hundreds to thousands and matched for age, gender and optionally other variables of interest. The normal range in terms of absolute values, like a concentration given, also depends on the assay employed and the standardization used in producing the assay.

The levels given for the hemoglobin/haptoglobin complex and calprotectin in the examples section have been measured from aliquots derived from the same stool sample and established with the assay procedures given.

A "marker" according to the present invention always relates to a polypeptide form of the mentioned biomolecule, i.e. not to the DNA nor to the mRNA coding for such marker.

In a method according to the present invention at least the concentration of each of the biomarkers hemoglobin/haptoglobin complex and calprotectin, respectively, as present in a stool sample is determined the measured values are mathematically combined and the combined value of the marker combination is correlated to the absence or presence of CRC.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. be the case when diagnosing an infectious disease, like AIDS. Frequently, however, the combination of markers is evaluated. In the method according to the present invention, the values measured for markers of a marker panel, e.g. for the hemoglobin/haptoglobin complex and calprotectin, are mathematically combined and the combined value is correlated to the underlying diagnostic question.

Marker values may be combined by any appropriate state of the art mathematical method. Well-known mathematical methods for correlating a marker combination to a disease employ methods like, Discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of CRC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al., J. of Computational and Graphical Statistics 12 (2003) 475-511; Friedman, J.H., J. of the American Statistical Association 84 (1989) 165-175; Hastie, T., et al., The Elements of Statistical Learning, Springer Verlag (2001); Breiman, L., et al., Classification and regression trees, California, Wadsworth (1984); Breiman, L., Machine Learning 45 (2001) 5-32; Pepe, M.S., The Statistical Evaluation of Medical Tests for Classification and Prediction, Oxford Statistical Science Series, 28 (2003); and Duda, R.O., et al., Pattern Classification, Wiley Interscience, 2nd edition (2001).

It is a preferred embodiment of the invention to use an optimized multivariate cut-off for the underlying combination of biological markers and to discriminate state A from state B, e.g. presence of CRC from absence of CRC. In this type of analysis the markers are no longer independent but form a marker panel. It could be established that combining the measurements of the hemoglobin/haptoglobin complex and of calprotectin does improve the diagnostic accuracy for CRC as compared to healthy controls. This becomes especially evident if only samples obtained from patients with early stages of CRC (UICC stages I to II) are included in the analysis. Especially the later finding is of great importance, because patients with early CRC are likely to profit most from a correct and early detection of a malignancy.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In .each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test result)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive result)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot (area under the curve = AUC). By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

In a preferred embodiment the present invention relates to a method for improving the diagnostic accuracy for colorectal cancer, i.e. patients suffering from CRC, versus controls, i.e. patients without CRC, by measuring in a sample the concentration of at least the hemoglobin/haptoglobin complex and calprotectin and correlating the concentrations determined to the presence or absence of CRC. This results in an improvement with more patients being correctly classified as suffering from CRC versus healthy controls as compared to a classification based on either marker alone. The CRC marker panel comprising the hemoglobin/haptoglobin complex and calprotectin can of course also be used in assessing the severity of disease for patients suffering from CRC.

As the skilled artisan will appreciate one or more additional biomarker may be used to further improve the assessment of CRC. To illustrate this additional potential of using the hemoglobin/haptoglobin complex and calprotectin as the key markers of a panel of markers for assessment of CRC the term "at least" has been used in the appending claims. With other words, the level measured for one or more additional marker may be combined with the measurement of the hemoglobin/haptoglobin complex and of calprotectin in the assessment of CRC.

The one or more additional marker used together with the hemoglobin/haptoglobin complex and calprotectin may be considered to be part of a CRC marker panel, i.e., a series of markers appropriate to further refine the assessment of CRC. The total number of markers in a CRC marker panel is preferably less than 20 markers, more preferred less than 15 markers, also preferred are less than 10 markers with 8 or less markers being even more preferred. Preferred are CRC marker panels comprising 3, 4, 5, or 6 markers in total.

In a preferred embodiment the present invention thus relates to a method for assessing the absence or presence of colorectal cancer in vitro by biochemical markers, comprising measuring in a sample the concentration of the hemoglobin/haptoglobin complex and of calprotectin and in addition the concentration of one or more other marker and correlating the concentrations of the hemoglobin/haptoglobin complex, calprotectin, and of the one or more additional marker to the absence or presence of colorectal cancer.

Preferably the one or more other marker is selected from the group consisting of CEA, CYFRA 21-1, CA19-9, CA72-4, NNMT, PROC, and SAHH.

An assay for "CYFRA 21-1" specifically measures a soluble fragment of cytokeratin 19 as present in the circulation. The measurement of CYFRA 21-1 is typically based upon two monoclonal antibodies (Bodenmueller, H., et al., Int. J. Biol. Markers 9 (1994) 75-81). In the CYFRA 21-1 assay from Roche Diagnostics, Germany, the two specific monoclonal antibodies (KS 19.1 and BM 19.21) are used and a soluble fragment of cytokeratin 19 having a molecular weight of approx. 30,000 Daltons is measured.

The Carbohydrate Antigen 19-9 (CA 19-9) values measured are defined by the use of the monoclonal antibody 1116-NS-19-9. The 1116-NS-19-9-reactive determinants on a glycolipid having a molecular weight of approx. 10,000 Daltons are measured. This mucin corresponds to a hapten of Lewis-a blood group determinants and is a component of a number of mucous membrane cells. (Koprowski, H., et al., Somatic Cell Genet. 5 (1979) 957-972). CA 19-9 can, e.g., be measured on the Elecsys® analyzer using Roche product number 11776193 according to the manufacturers instructions.

The CA 72-4 assay determines the mucine-like tumor-associated glycoprotein TAG 72 in serum using two monoclonal antibodies. The monoclonal B72.3 has been raised against a membrane-enriched extract of of mammary carcinoma metastases (Colcher, D., et. al., Proc. Natl. Acad. Sci. 78 (1981) 3199-3203). The monoclonal CC49 is specific to highly purified TAG 72. CA 72-4 can be measured on the Elecsys® analyzer using Roche product number 11776258 according to the manufacturer's instructions.

Carcinoembryonic antigen (CEA) is a monomeric glycoprotein (molecular weight approx. 180.000 Dalton) with a variable carbohydrate component of approx. 45-60 % (Gold, P. and Freedman S. O., J. Exp. Med. 121 (1965) 439-462). High CEA concentrations are frequently found in cases of colorectal adenocarcinoma (Fateh-Moghadam, A., and Stieber, P., Sensible use of tumor markers, Boehringer Mannheim, Cat. No. 1536869 (Engl.), 1320947 (German), ISBN 3-926725-07-9 German/English, Juergen Hartmann Verlag GmbH, Marloffstein-Rathsberg (1993). Slight to moderate CEA elevations (rarely > 10 ng/mL) occur in 20-50 % of benign diseases of the intestine, the pancreas, the liver, and the lungs (e.g. liver cirrhosis, chronic hepatitis, pancreatitis, ulcerative colitis, Crohn's Disease, emphysema) (Fateh-Moghadam, A., and Stieber, P., supra). Smokers also have elevated CEA values. The main indication for CEA determinations is the follow-up and therapy management of colorectal carcinoma.

The protein nicotinamide N-methyltransferase (NNMT; Swiss-PROT: P40261) has an apparent molecular weight of 29.6 kDa and an isoelectric point of 5.56. It has recently been found (WO 2004/057336) that NNMT will be of interest in the assessment of CRC. The immunoassay described in WO 2004/057336 has been used to measure the samples (CRC, healthy controls and non-malignant colon diseases) of the present study.

The protein pyrroline-5-carboxylate reductase (PROC; Swiss-PROT: P32322) is also known as PYCR1 in the literature. PROC catalyzes the NAD(P)H-dependent conversion of pyrroline-5-carboxylate to proline. Merrill, M. J., et al., J. Biol. Chem. 264 (1989) 9352-9358 studied the properties of human erythrocyte pyrroline-5-carboxylate reductase. They concluded that in addition to the traditional role of catalyzing the obligatory and final unidirectional step in pyrroline biosynthesis, the enzyme may play a physiologic role in the generation of NADP(+) in some cell types including human erythrocytes. PROC has recently been identified as a marker of CRC (WO 2005/095978).

The protein SAHH (S-adenosylhomocysteine hydrolase; SWISS-PROT: P23526) has recently been identified as a marker of colorectal cancer (WO 2005/015221). The corresponding cloned human cDNA encodes for a 48-kDa protein. SAHH catalyzes the following reversible reaction: S-adenosyl-L-homocysteine + H2O ↔ adenosine + L-homocysteine (Cantoni, G. L., Annu. Rev. Biochem. 44 (1975) 435-451). Hershfield and Francke (Hershfield, M.S. and Francke, U., Science 216 (1982) 739-742) located the corresponding gene to chromosome 20 and later on Coulter-Karis and Hershfield (Coulter-Karis, D.E. and Hershfield, M.S., Ann. Hum. Genet. 53 (1989) 169-175) sequenced the full-length cDNA. Recently, the structure of SAHH has been resolved (Turner, M.A., et al., Cell. Biochem. Biophys. 33 (2000) 101-125).

As the skilled artisan will appreciate one or more additional marker may be used to further improve the diagnostic accuracy, or, where required increase the diagnostic sensitivity at the expense of specificity or vice versa. In some diagnostic areas, e.g., in the detection of an HIV-infection sensitivity is of utmost importance. The high sensitivity required may be achieved at the expense of specificity, leading to an increased number of false positive cases. In other cases, e.g. as a simple example, when assessing blood group antigens, specificity is of paramount importance.

The method according to the present invention appears to be suitable for screening asymptomatic individuals for the presence or absence of CRC. In doing so, both specificity as well as sensitivity are of paramount importance. It is generally accepted that a method used in the screening for a disease with low prevalence, like CRC, the specificity has to be at least 90%, preferably even 95%, and also preferably even 98% or 99%. With other words, in the latter case the false positive fraction would be 2% or less, or 1% or less, respectively. This means that not too many costly follow-up examinations are inadvertently caused (due to a false positive result) at such a high level of specificity. Preferably the method for assessing the absence or presence of colorectal cancer in vitro by biochemical markers according to the present invention has a specificity of at least 90%, even more preferred of at least 95%. Also preferably, the specificity is at least 98% or at least 99%, respectively.

The method for assessing the absence or presence of colorectal cancer in vitro by measuring at least the hemoglobin/haptoglobin complex and calprotectin in a stool sample according to the present invention has an improved sensitivity for detection of CRC both at a fixed level of specificity of 95% and of 98%, respectively.

A further preferred embodiment relates to the use of a marker panel in the diagnosis of CRC the panel comprising the hemoglobin/haptoglobin complex and calprotectin. Further preferred is the use of a marker panel comprising the hemoglobin/haptoglobin complex, calprotectin, and at least one additional polypeptide marker selected from the group consisting of CEA, CYFRA 21-1, CA19-9, CA72-4, NNMT, PROC, and SAHH.

In a preferred embodiment the method according to the present invention for assessing the absence or presence of colorectal cancer in vitro by biochemical markers that comprises measuring in a stool sample the concentration of at least the hemoglobin/haptoglobin complex and calprotectin makes use of a special new diluent for stool samples described in some detail below.

A preferred stool sample diluent will at least comprise a buffer, a protease inhibitor, and a analyte-releasing reagent without being restricted to e.g. detergents or chaotropic reagents. The buffer in certain preferred embodiments additionally comprises a blocking agent and/or a preservative.

The skilled artisan is familiar with appropriate buffer systems. Preferably the buffer or buffer system will be selected from the group consisting of phosphate buffered saline (PBS), Tris- Hydroxymethylaminoethane (Tris) buffered saline (TBS), N-(2-hydroxyethyl)-piperazine-N'-2-ethanesulfonic acid (HEPES), and 3-(N-Morpholino) propanesulfonic acid (MOPS). Preferably the buffer will have a molarity of between 20 and 200 mM.

The pH of the stool sample diluent preferably is adjusted to a pH-value between pH 6.5 and pH 8.5, more preferably to a pH-value between pH 7.0 and pH 8.0, and further preferred to a pH-value between pH 7.2 and pH 7.7. The skilled artisan will have no difficulty in selecting the appropriate concentration of the buffer constituents in order to ensure that after diluting and mixing the stool specimen with the stool sample diluent the desired pH is attained.

The stool sample diluent comprises a protease inhibitor. There is an ever increasing number of proteases and also of corresponding protease inhibitors.

One important class of proteases are the so-called serine proteases that have the amino acid serine in their active site. Well-known examples of serine proteases are trypsin, chymotrypsin, kallikrein, and urokinase. The skilled artisan is familiar with the fact that certain protease inhibitors are active against serine proteases. The inhibitory potential of such proteases and their activity spectrum is e.g. described in the data sheets from commercial suppliers, like Serva, Heidelberg, or Roche Diagnostics GmbH, Mannheim. Preferably the serine protease inhibitor is selected from the group consisting of AEBSF-HCl (e.g., Serva Cat.No. 12745), APMSF-HCl (e.g., Serva Cat.No. 12320), aprotinin (e.g., Roche Diagnostics, Cat.No. 10 981 532 001), chymostatin (e.g., Roche Diagnostics, Cat.No. 11 004 638 001), Pefabloc ® SC (e.g., Roche Diagnostics, Cat.No. 11 585 916 001), and PMSF (e.g., Roche Diagnostics, Cat.No. 10 837 091 001).

A further important class of proteases are the so-called cysteine proteases that have the amino acid cysteine in their active site. Well-known examples of cysteine proteases are papain and calpain. The skilled artisan is familiar with the fact that certain protease inhibitors are active against cysteine proteases. Some of these inhibitors are also active against serine proteases, e.g., PMSF may be used as an inhibitor of cysteine proteases as well as an inhibitor of serine proteases. The inhibitory potential of such proteases and their activity spectrum is e.g. described in the data sheets from commercial suppliers, like Serva, Heidelberg, or Roche Diagnostics GmbH, Mannheim. Preferably the cysteine protease inhibitor is selected from the group consisting of leupeptine(e.g., Roche Diagnostics, Cat.No. 11 034 626 001), PMSF (see above), and E-64 (e.g., Roche Diagnostics, Cat.No. 10 874 523 001).

A further important class of proteases are the so-called metalloproteases. Metalloproteases are characterized by containing a metal ion e.g., Zn²⁺, Ca²⁺ or Mn²⁺ in the active center. Well-known examples of metalloproteases are digestive enzymes such as carboxypeptidases A and B and thermolysin. The skilled artisan is familiar with the fact that certain protease inhibitors are active against metalloproteases. Metalloproteases are most easily inactivated by substances binding to the metal ion and forming a metal chelate complex therewith. Preferably ethylene-diaminotetra acetic acid (EDTA), ethylene glycol bis(aminoethylether)tetra acetic acid (EGTA), and/or 1,2-diaminocyclohexane-N,N,N',N'-tetra acetic acid (CDTA) are used to inactivate metalloproteases. Other appropriate inhibitors of metalloproteases are Phosphoramidon (= N-(α-Rhamnopyranosyloxyhydroxyphosphinyl)-L-leucyl-Ltryptophan, disodium salt; e.g., Roche Diagnostics Cat.No. 10 874 531 001) and bestatin (e.g., Roche Diagnostics Cat.No. 10 874 515 001). The inhibitory potential of these protease inhibitors and their activity spectrum is e.g. described in the corresponding data sheets from commercial suppliers, like Serva, Heidelberg, or Roche Diagnostics GmbH, Mannheim. Preferred inhibitors of metalloproteases are EDTA, EGTA and/or bestatin.

A further important class of proteases is known as aspartic (acidic) proteases. Aspartic proteases are characterized by having an aspartic acid residue in the active center. Well-known examples of aspartic proteases are pepsin, cathepsin D, chymosin, and renin. The skilled artisan is familiar with the fact that certain protease inhibitors are active against aspartic proteases. Preferred inhibitors of aspartic acid proteases are α2-macroglobulin (e.g., Roche Diagnostics Cat.No. 10 602 442 001) and pepstatin (e.g., Roche Diagnostics Cat.No. 11 359 053 001).

For certain applications it will be possible to apply the method according to the present invention by using a stool sample diluent that comprises only one protease inhibitor that protects the polypeptide of interest by e.g. blocking a certain class of proteases.

Preferably the stool sample diluent will comprise at least two different protease inhibitors with activity against two classes of proteases selected from the group consisting of serine proteases, cysteine proteases, metalloproteases and aspartic proteases. Also preferred at least three of these enzyme classes will be inhibited by an appropriate inhibitor cocktail. Preferably the stool sample diluent will contain a protease inhibitor cocktail that is composed of protease inhibitors that are active against serine proteases, cysteine proteases, metalloproteases and aspartic proteases, respectively.

Preferably at most 20 different protease inhibitors will be used to set up a protease inhibitor cocktail for a stool sample diluent. Also preferred no more than 15 different protease inhibitors will be used. Preferably 10 or less different protease inhibitors as contained in a stool diluent, will suffice to achieve sufficient protease inhibition in order to stabilize a proteinaceous analyte in a stool sample.

Preferably the protease inhibitor is selected from the group consisting of aprotinin, chymostatin, leupeptine, EDTA, EGTA, CDTA, pepstatin A, phenylmethylsulfonyl fluoride (PMSF), and Pefabloc^{®} SC. Preferably the protease inhibitor cocktail contains chymostatin, leupeptine, CDTA, pepstatin A, PMSF, and Pefabloc^{®} SC, also preferred a protease inhibitor cocktail containing aprotinin, leupeptine, EDTA and Pefabloc^{®} SC is used.

A preferred stool sample diluent also comprises a detergent and/or a chaotropic reagent. Detergents are usually classified into anionic detergents, cationic detergents, amphiphilic detergents and nonionic detergents. The detergent and/or chaotropic reagent optimal for use in a stool sample diluent according to the present invention must be capable of releasing the analyte of interest from the sample and at the same time it should allow for stabilization of the analyte. This tightrope walk surprisingly can be accomplished by use of different detergents or chaotropic reagents. Preferably the analyte-releasing reagent used in a stool sample diluent according to the present invention is selected from the group of nonionic detergents like Brij 35®, Tween 20®, Thesit®, Triton X100®, and Nonidet P40 and/or from the group of chaotropic reagents like urea, SCN, guadinium-hydrochlride etc. Most procedures using a stool specimen as a sample require the direct transfer of the stool specimen to the test system, e.g. to the test areas of a guaiac test. This procedure reduces the compliance rate of thr patients.

The less handling steps and the more robust the sampling and extraction of a stool sample the better.

Several recent developments have focused on device that facilitate the sampling and handling of a stool sample. EP 1 366 715 discloses a special collection tube for collection of a stool sample. This extraction tube essentially comprises (a) a container body that is hollow on the inside, open at the top, and able to receive a buffer solution, (b) a top cap provided with a threaded small rod for collection of fecal samples, said threaded small rod protruding axially inside the container body, when the top cap is applied to the top end of the container body, and (c) a dividing partition provided, in an intermediate position, inside said container body so as to separate a top chamber from a bottom chamber inside said container body, said dividing partition having an axial hole suitable to allow the passage of said threaded small rod, so as to retain the excess feces in said top chamber and allow the passage of the threaded part of the small rod into said bottom chamber. This extraction tube further has a container body that is open at the bottom and provided with a bottom cap which can be applied movably to the bottom end of the container body, so that said extraction tube can be used directly as a primary sampling tube to be inserted into a sample-holder plate of automatic analyzers, following removal of said bottom cap and overturning of said container body. With more simple words the tube disclosed in EP 1 366 715 allows for a convenient handling of a defined quantity of a stool sample and has the advantage that after appropriate extraction the tube may be directly placed into the sample-holder of an automatic analyzer. The reader will find the detailed disclosure of this stool sampling tube in the above captioned patent, the full disclosure is herewith incorporated by reference.

In WO 03/068398 a sophisticated stool sampling device is described that also is appropriate for a convenient sampling and handling of a stool sample. The features of the device disclosed in this WO-application are explicitly referred to and herewith enclosed by reference in their entirety. In WO 03/069343 it is recommended to extract a stool specimen, e.g., collected with a device according to WO 03/068398 by use of an extraction buffer comprising 10 mM CHAPS (= 3-[(3-chloramidopropyl)-dimethylammonio]-1-propanesulfonate), which is a zwitterionic detergent.

For preparing a fecal sample composition for immuno assay testing a dispersion of at most 10 wt. %, preferably from 0.1 wt. % to up to 10 wt. % and more preferably from 0.5 to 5 wt. % of a stool sample in the stool sample diluent is made. Preferably the mixing of the stool sample with the diluent is made directly within an appropriate sampling tube already prefilled with a stool sample diluent as described above.

The stool sample is preferably freshly collected and given into the stool sample diluent directly. No intermediate storage, transportation and/or handling is necessary.

The level of the hemoglobin/haptoglobin complex and calprotectin, respectively, is detected by any appropriate assay method. In clinical routine such methods in most cases will employ antibodies to the target antigen, the so-called immuno assays. A wide variety of immuno assay procedures including latex agglutination, competition and sandwich immuno assays can be carried out for detecting a proteinaceous analyte in a stool sample if such stool sample is e.g., prepared as described in detail above.

The immuno assay used preferably is a heterogeneous immuno assay. It is also preferred that the detection of the proteinaceous analyte is accomplished by aid of a competitive immuno assay or by aid of a so-called sandwich immuno assay.

The skilled artisan will have no problem in setting up an immuno assay which is capable of detecting the target antigen or target analyte as present in the extract of a stool sample.

By way of example such detection may be performed in a sandwich type immuno assay. Typically a first anti-analyte antibody is directly or indirectly bound to a solid phase. With other words, the first antibody binding to the target antigen is used as a capture antibody. For determining a target analyte, e.g. in an extract of a human stool sample the extract is incubated under appropriate conditions and for a time sufficient to permit a binding of the capture antibody to the analyte. For detection of the target antigen a second or detection antibody to the target antigen which binds to an epitope different to the one recognized by the capture antibody is used. Incubation with this second antibody may be performed before, after or at the same time as the incubation with the first antibody.

Preferably the detection antibody is labeled in such a manner that direct or indirect detection is facilitated.

For direct detection the labeling group can be selected from any known detectable marker groups, such as dyes, luminescent labeling groups such as chemiluminescent groups, e.g., acridinium esters or dioxetanes, or fluorescent dyes, e.g., fluorescein, coumarin, rhodamine, oxazine, resorufin, cyanine and derivatives thereof. Other examples of labeling groups are luminescent metal complexes, such as ruthenium or europium complexes, enzymes, e.g., as used for ELISA or for CEDIA (Cloned Enzyme Donor Immuno assay, e.g., EP 0 061 888), and radioisotopes.

Indirect detection systems comprise, for example, that the detection reagent, e.g., the detection antibody is labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g., steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is labeled to allow for direct detection, e.g., by the labels as mentioned above.

Immuno assays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates, In: Practice and theory of enzyme immunoassays, Burdon, R.H. and v. Knippenberg, P.H. (eds.), Elsevier, Amsterdam (1990), pp. 221-278), and various volumes of Methods in Enzymology, Colowick, S.P. and Caplan, N.O. (eds.), Academic Press), dealing with immunological detection methods, especially volumes 70, 73, 74, 84, 92 and 121.

Based on the stool sample diluent described above, it is possible to handle a stool sample in a very convenient manner. Preferably at least one of the markers hemoglobin/haptoglobin complex and calprotectin is detected from a stool sample collected in a stool sample diluent as described above. Preferably both analytes are detected from a stool sample collected in a stool sample diluent as described above. It is also preferred to use the preferred compositions of such a stool sample diluent in the detection of either calprotectin or the hemoglobin/haptoglobin complex, or in the detection of both these analytes.

The present invention also relates to a kit for performing the method of this invention comprising the reagents required to specifically measure the hemoglobin/haptoglobin complex and calprotectin, respectively.

In yet a further preferred embodiment the kit will comprise reagents required for performing the measurement of both the hemoglobin/haptoglobin complex and calprotectin and in addition a stool sampling device, prefilled with an appropriate stool sample diluent.

The following examples are provided to aid the understanding of the present invention, the true scope of which is set forth in the appended claims. It is understood that modifications can be made in the procedures set forth without departing from the spirit of the invention.

### Example 1

### Processing of stool specimen

To improve the measurement of different analytes of interest in stool samples an "optimized extraction buffer" is used. For the processing of the stool samples the extraction buffer is freshly prepared by adding a protease inhibitor cocktail (Mini Complete EDTA-free, Roche, Germany) to the following buffer:

| | |
|---|---|
| TRIS | 0.10 mol/l, pH 8.0 |
| Citric acid | 0.10 mol/l |
| Urea | 1.0 mol/l |
| CaCl₂ | 0.01 mol/l |
| BSA | 0.50 % |

The stool samples are thawed and 50 - 100 mg of each sample are transferred to a fecal sample preparation kit (cat.-no.: 10745804 Roche, Germany). Optimized extraction buffer is added according to the weight of the stool samples to give a 50-fold dilution. The samples are vigorously mixed on an orbital shaker for 30 minutes, transferred to a 10 ml tube (Sarstedt, Germany) and centrifuged at 1200 g for 10 minutes. The supernatant is filtered using a 5 µm cut-off filter (Ultrafree®-CL, Millipore, Germany), aliquoted and stored for further analysis at -70 °C. These stool extracts are suitable for all biomarkers of interest in this study.

### Example 2

### Analyte stability in stool extract

Calprotectin appears to be more stable than hemoglobin-haptoglobin in stool extracts prepared using the extraction method described in example 1. When stool extracts are stored for 1 or 3 days at room temperature the average recovery for Calprotectin is higher and appears to show less scatter than the average recovery of hemoglobin-haptoglobin. Of the 20 samples used to assess the stability 5 were hemoglobin-haptoglobin negative:

**Table 1: Recovery after temperature stress**

| | **N** | **Concentration range of samples** | **Recovery after 1 d RT** | **Recovery after 3 d RT** |
|---|---|---|---|---|
| Calprotectin | 20 | 18 - 6250 µg/g | 96 % (± 10 %) | 94 % (± 19%) |
| Hb-Hp | 15 | 0.2 - 4790 µg/g | 78 % (± 33 %) | 72 % (± 30 %) |

### Example 3

### Clinical utility study in colorectal cancer

The clinical utility is assessed by analyzing stool samples obtained from well-characterized patient cohorts. For each patient two stool samples from the same bowel movement are measured and the concentrations are analyzed. To improve the sensitivity of the assay the maximum concentration measured in one of the two paired samples is used for further analysis. The diagnostic value of Calprotectin and hemoglobnin-haptoglobin and their combination is also evaluated by ROC analysis according to Zweig et al. (supra).

### Study population

All analytes are measured in a large study population (for patient characteristics cf.: Table 2). A high number of clinically well-characterized stool samples is prospectively collected in the frame-work of multi-center study. The patients (undergoing a colonoscopy) for the control collective are recruited at gastroenterology units and representing an average-risk screening population. Patients with inflammatory bowel diseases and with any kind of adenoma are excluded from the control collective. Due to the low prevalence of colorectal cancer patients in a screening population, the samples from cancer patients are collected at different surgery units. The diagnosis of colorectal cancer is confirmed by a physician also providing the pathological staging for each cancer patient. To exclude any bias that might be introduced by the common diagnostic work-up of patients by a guaiac-based FOBT prescreening, all CRC patients primarily detected due to visible rectal bleeding or due to a positive FOBT are excluded from this collective. Because these would introduce a bias to the advantage of hemoglobin.

Stool samples are obtained from 252 control individuals. Of these 132 are confirmed by colonoscopy to be GI-healthy, while the remaining control samples cover several relevant GI-diseases. The CRC population includes 101 CRC samples from UICC stages I - IV (Table 3). For 16 CRC patients the exact staging is not known.

**Table 2: Patient characteristics of the study population**

| | **Total number** | **Age (year)** | **Gender** |
|---|---|---|---|
| | | | **(female/male)** |
| Controls | 252 | 63,0 +/- 8,0 | 151/101 |
| - Healthy Controls (no evidence of any bowel disease) | 132 | 62,3 +/- 6,8 | 81/51 |
| - Hemorrhoids | 28 | 60,1 +/- 7,1 | 13/15 |
| - Diverticulosis | 73 | 64,7 +/- 9,5 | 46/27 |
| - Hyplastic polyps | 14 | 67,9 +/- 9,9 | 8/6 |
| - Other GI diseases | 5 | 59,2 +/- 6,7 | 3/2 |
| CRC | 101 | 68,4 +/- 11,5 | 48/53 |

**Table 3: Distribution of CRC UICC stages**

| **Sample panel** | **N** |
|---|---|
| CRC | **101** |
| UICC stage 0/I | **23** |
| UICC stage II | **27** |
| UICC stage III | **12** |
| UICC stage IV | **23** |
| Without staging | **16** |

### Combination of Calprotectin, hemoglobin-haptoglobin and other stool markers

Combinations of Calprotectin with other biomarkers from stool extracts are evaluated. The markers Calprotectin, hemoglobin, the hetero-complex of hemoglobin with haptoglobin and CEA are measured using commercial ELISAs. Assays for measurement of hemoglobin, hemoglobin/ haptoglobin and Calprotectin are obtained from R-Biopharm, Germany. The Calprotectin ELISA is manufactured by Calpro SA, Norway, and is marketed outside of Germany as PhiCal^{™} Test. The assay for measurement of CEA is obtained from Roche Diagnostics, Germany. While some of the assays are intended for measurements in stool extracts, for CEA stool is not a commonly used sample material. Hence, the assay has to be adjusted for the measurement in extracted stool specimen. The samples (stool extracts) are prediluted 20-fold for CEA determinations, but otherwise the assay is run according to the manufacturers recommendations.

To test if a marker combination will improve the diagnosis of CRC, the markers are combined by Bayes Logistic Regression (BLR). In the BLR algorithm for the evaluation of marker combinations a Gaussian prior is used and implemented in the BBR-Software of Alexander Genkin, David D. Lewis, and David Madigan (Large-scale Bayesian logistic regression for text categorization, Technometrics). The following settings are used: no automatic feature selection, prior variance fixed at 0.05, no threshold-tuning, and input standardization by normalization. For the numerical process the default settings with a convergence threshold of 0.0005, 1000 iterations and no-accuracy-mode are retained unchanged. The results with the basic algorithm get evaluated by 100 runs in a Monte-Carlo cross-validation design. In each run, two-third of all cases and controls, respectively, are selected as training set via the Matlab® R2006a in-built function randsample with starting value 19022007 for the default random number generator. The basic algorithm is applied on the training set to generate a diagnostic rule. A threshold on the estimated posterior case-probabilities is determined on the controls of the training set to achieve a specificity of 95% or 98%, respectively. The diagnostic rule is then applied to the other third of the data to estimate sensitivity and specificity at the given threshold.

To avoid any bias for hemoglobin or hemoglobin / haptoglobin only CRC patients without prior FOBT prescreening are used in the assessment. For a screening assay not only the AUC of the ROC plot is relevant. A quite critical requirement in a screening setting is a good enough sensitivity at a high specificity. High specificity is crucial because a low specificity will cause a high number of false positive results accompanied by unnecessary follow-up procedures and distress for the patients. Table 4 summarizes the AUC values of the evaluation as well as the sensitivities at a preset specificity of 95% and 98%, respectively. When some of the individual markers measured are combined by BLR the highest AUC value is achieved by the combination of Calprotectin and hemoglobin-haptoglobin with 95% area under the curve. On the other hand the overall sensitivity in detection of CRC can be improved by combination of hemoglobin-haptoglobin with Calprotectin at both specificity levels of 95% and 98%. In both cases the sensitivity of the combination of Calprotectin with hemoglobin-haptoglobin is surprisingly higher than the combination of Calprotectin with hemoglobin. These marker combination can be considered very important in order to detect CRC, especially CRC at early stages.

**Table 4: Marker combinations for the detection of CRC**

| Marker combination | Hb-Hp | Calprotectin | Calprotectin + Hb-Hp | Calprotectin + Hb | Calprotectin + CEA |
|---|---|---|---|---|---|
| AUC % | 92 | 90 | 95 | 94 | 89 |
| Sensitivity at 95 % Spec. | 82 % | 62% | 85% | 82% | 64% |
| Sensitivity at 98 % Spec. | 73% | 55% | 76 % | 73% | 52 % |

## Claims

1. A method for assessing the absence or presence of colorectal cancer in vitro by biochemical markers, comprising measuring in a stool sample the concentration of at least
a) the hemoglobin-haptoglobin complex and
b) calprotectin, and
c) correlating the concentrations determined in steps a) and b) to the absence or presence of colorectal cancer.

2. The method according to claim 1, further comprising the measurement of at least one additional polypeptide marker selected from the group consisting of CEA, CYFRA 21-1, CA19-9, CA72-4, NNMT, PROC, and SAHH.

3. Use of a marker panel comprising at least the hemoglobin/haptoglobin complex and calprotectin in the diagnosis of colorectal cancer.

4. Use according to claim 3 comprising the hemoglobin/haptoglobin complex, calprotectin and at least one additional marker selected from the group consisting of CEA, CYFRA 21-1, CA19-9, CA72-4, NNMT, PROC, and SAHH.

5. A kit for performing the method according to claim 1 comprising the reagents required to specifically measure the hemoglobin/haptoglobin complex and calprotectin, respectively, and optionally auxiliary reagents for performing the measurement.

## Patentansprüche

1. In-vitro-Verfahren zur Beurteilung des Fehlens oder Vorliegens eines Kolorektalkarzinoms mittels biochemischer Marker, wobei man in einer Stuhlprobe die Konzentration zumindest
a) des Hämoglobin-Haptoglobin-Komplexes und
b) von Calprotectin misst und
c) die in Schritt a) und b) bestimmten Konzentrationen mit dem Fehlen bzw. Vorliegen eines Kolorektalkarzinoms korreliert.

2. Verfahren nach Anspruch 1, bei dem man ferner wenigstens einen zusätzlichen Polypeptidmarker, ausgewählt aus der Gruppe bestehend aus CEA, CYFRA 21-1, CA19-9, CA72-4, NNMT, PROC und SAHH, misst.

3. Verwendung einer Markergruppe, umfassend wenigstens den Hämoglobin/Haptoglobin-Komplex und Calprotectin, bei der Kolorektalkarzinom-Diagnose.

4. Verwendung nach Anspruch 3, umfassend den Hämoglobin/Haptoglobin-Komplex, Calprotectin sowie einen zusätzlichen Marker, ausgewählt aus der Gruppe bestehend aus CEA, CYFRA 21-1, CA19-9, CA72-4, NNMT, PROC und SAHH.

5. Kit zur Ausführung des Verfahrens nach Anspruch 1, umfassend die zur spezifischen Messung des Hämoglobin-Haptoglobin-Komplexes bzw. von Calprotectin benötigten Reagentien sowie gegebenenfalls Hilfsreagentien zur Ausführung der Messung.

## Revendications

1. Procédé pour évaluer l'absence ou la présence d'un cancer colo-rectal in vitro via des marqueurs biochimiques, comprenant la mesure, dans un échantillon de selles, de la concentration d'au moins :
a) le complexe hémoglobine-haptoglobine, et
b) la calprotectine, et
c) la mise en corrélation des concentrations déterminées aux étapes a) et b) avec l'absence ou la présence d'un cancer colo-rectal.

2. Procédé selon la revendication 1, comprenant en outre la mesure d'au moins un marqueur polypeptidique supplémentaire choisi parmi le groupe constitué par CEA, CYFRA 21-1, CA19-9, CA72-4, NNMT, PROC et SAHH.

3. Utilisation d'un panel de marqueurs comprenant au moins le complexe d'hémoglobine/haptoglobine et la calprotectine dans le diagnostic du cancer colo-rectal.

4. Utilisation selon la revendication 3, comprenant le complexe d'hémoglobine/haptoglobine, la calprotectine et au moins un marqueur supplémentaire choisi parmi le groupe constitué par CEA, CYFRA 21-1, CA19-9, CA72-4, NNMT, PROC et SAHH.

5. Nécessaire pour la mise en oeuvre du procédé selon la revendication 1, comprenant les réactifs requis pour mesurer de manière spécifique le complexe d'hémoglobine/haptoglobine et la calprotectine, respectivement, et de manière facultative, des réactifs auxiliaires pour la mise en oeuvre de la mesure.
